# FASCICULE DE BREVET EUROPEEN

(11) **EP 0 445 246 B1**
(45) Date de publication et mention de la délivrance du brevet: **02.08.1995**
(21) Numéro de dépôt: 90913459.5
(22) Date de dépôt: 26.09.1990
(51) Int. Cl.: A61B 10/00, A61B 19/00, A61B 17/30

(54) **INSTRUMENT POUR LA MANUTENTION DE BIOPSIES ET PRELEVEMENTS**
VORRICHTUNG ZUR HANDHABUNG VON BIOPSIEN UND PROBEN
INSTRUMENT FOR HANDLING BIOPSIES AND SAMPLES

(30) Priorité: 27.09.1989 CH 3504/89; 06.08.1990 CH 2557/90
(43) Date de publication de la demande: 11.09.1991
(73) Titulaire: BREDAM MEDICAL DISTRIBUTION S.A., CH-1701 Fribourg (CH)
(72) Inventeur: GILLIARD, René, CH-1025 Saint-Sulpice (CH)
(74) Mandataire: Ganguillet, Cyril
(86) Numéro de dépôt international: CH9000228
(87) Numéro de publication internationale: WO9104709

(56) Documents cités:
- CH-A- 659 382
- GB-A- 280 495
- US-A- 3 515 128
- US-A- 3 938 505
- US-A- 4 549 554

## Description

La présente invention concerne un instrument pour la manutention de biopsies et prélèvements.

Lorsqu'un médecin tient à connaître la nature d'un tissu, il fait en général un ou plusieurs prélèvements et les fait ensuite analyser.

Quelle que soit en fait la discipline médicale concernée, on rencontre le même type de problèmes lorsqu'il s'agit de manipuler les prélèvements et biopsies. En effet, les prélèvements et biopsies ont en général très peu de consistance et se présentent comme de petites masses collantes qui adhèrent obstinément à l'instrument qui permet de les réaliser. Selon les domaines, la manipulation est rendue encore plus difficile en raison de la très petite taille des instruments de prélèvement et par conséquent des prélèvements eux-mêmes.

D'une manière générale, la première phase de la manipulation consiste à détacher le prélèvement de l'instrument pour le déposer dans un flacon contenant un liquide de conservation, flacon qui sera ensuite expédié au laboratoire d'analyse. Cette première opération se fait en général alors que le patient est présent et le médecin réutilise dans la plupart des cas le même instrument pour effectuer d'autres prélèvements sur le même patient. Dans ces conditions il n'est pas possible de tremper l'instrument de prélèvement dans le liquide de conservation et de l'y agiter pour que le prélèvement se détache de lui-même de l'instrument. En effet, il est exclu de réutiliser un instrument souillé du liquide de conservation. De plus, le liquide de conservation attaquerait l'instrument et le rendrait rapidement inutilisable.

La manipulation consiste donc par nécessité à tenir l'instrument contenant ou portant le prélèvement au dessus du flacon et à essayer de faire tomber le prélèvement dans le flacon. Pour ce faire, l'opérateur utilise un instrument pointu et décolle le prélèvement en grattant ou raclant celui-ci. Ce mode de procéder comporte des inconvénients de plusieurs ordres. En premier lieu, le prélèvement est souvent endommagé, à tel point quelquefois que l'analyse n'est même plus possible. Un deuxième inconvénient est que le tranchant de l'instrument, qui est évidemment primordial, supporte mal ce traitement. Un troisième inconvénient est que le médecin doit en général attendre que cette opération soit terminée et qu'on lui rende son instrument pour effectuer d'autres prélèvements. Le patient, souvent placé dans une situation peu confortable n'apprécie pas particulièrement cette attente forcée. Dans ces deux derniers cas, plus le prélèvement est petit ou collant, plus l'opération de transfert dans le flacon est difficile et plus l'attente est longue.

Le même type de problèmes est rencontré également dans les laboratoires d'analyses où les prélèvements font aussi l'objet de manipulations et de transferts, avec les mêmes moyens et des inconvénients similaires.

Dans le domaine médical, bon nombre d'opérations impliquant l'aspiration d'un liquide ou d'un tissu sont réalisées au moyen de seringues.

Certains modèles de seringues comportent un moyen de rappel, un ressort par exemple, tendant à ramener le piston dans une position de repos correspondant au volume enfermé maximum. On citera comme exemple le dispositif illustré dans le document US-A-4,664,128.

L'utilisation de seringues pour des opérations particulières, des ponctions de tissu par exemple, a également conduit à prévoir des mécanismes additionnels destinés à faciliter soit l'aspiration du tissu soit son éjection. En fait, le problème principal rencontré porte sur l'éjection du tissu après qu'il ait pénétré dans la seringue. Dans le document US-A-4,549,554, on a ainsi proposé d'aménager une prise d'air latérale dans le corps de la seringue, prise d'air dont l'ouverture et la fermeture sont commandées soit par un obturateur soit par la position relative du piston dans le cylindre de la seringue. La manoeuvre de l'obturateur, respectivement le déplacement du piston, permettent de laisser une quantité d'air entrer dans la seringue pour permettre l'éjection du tissu après que l'obturateur soit refermé ou que l'on ait repoussé de piston de manière qu'il masque à nouveau l'ouverture.

Cependant, aucune des solutions connues ne permet de résoudre, sans manipulation additionnelle, le problème du réarmement du volume d'air enfermé en vue de l'éjection du tissu. En d'autres termes, il n'existe pas dans l'état de la technique un dispositif qui résolve le problème de manière permanente par ses propres caractéristiques constructives, c'est à dire en ne recourant à aucune autre manipulation que l'opération élémentaire consistant à pousser avec le doigt sur la tige de commande du piston.

La présente invention a pour but de proposer un instrument qui permet de remédier aux inconvénients mentionnés plus haut.

A cet effet, l'invention concerne un instrument pour la manutention de biopsies et prélèvement, tel que décrit dans la revendication 1.

Avant d'entamer la description des diverses variantes de l'instrument selon l'invention, il est opportun de situer les problèmes techniques particuliers qui résultent de la fonction même de l'instrument ainsi que du cadre dans lequel il est destiné à être utilisé. En effet, ces problèmes se retrouvent dans la plupart des variantes exposées ci-après.

En premier lieu, il convient de noter que l'utilisateur tient en général un autre instrument dans l'une de ses mains. En conséquence, l'instrument de transfert doit pouvoir être actionné d'une main seulement. Cela signifie aussi que l'opérateur doit pouvoir commander, d'une main et sans difficulté, l'enchaînement de l'effet de succion et de l'effet d'éjection. En d'autres termes, l'instrument, qui est autonome par définition, doit permettre seul l'enchaînement de ces deux phases sans que le degré d'accomplissement de la première n'hypothèque la seconde.

Les diverses variantes de l'instrument selon l'invention sont décrites ci-dessous et les références indiquées se rapportent au dessin où:
- la figure 1: montre une version volontairement inachevée de l'instrument, afin d'illustrer par le dessin l'un des problèmes auxquels la réalisation de l'instrument est confrontée;
- la figure 2: illustre une vue schématique en perspective d'une première variante de l'instrument;
- la figure 3: illustre un détail, vu en coupe, de l'une des partie de l'instrument, sa partie destinée au contact avec le prélèvement;
- la figure 4: illustre en coupe un mode de réalisation particulier de la partie de contact entre l'instrument et le prélèvement;
- la figure 5: est une coupe de l'instrument selon une variante destinée à un usage répétitif;
- la figure 6: illustre les adjonctions qui peuvent être apportées à la variante de l'instrument destiné à un usage répétitif, pour parvenir à une deuxième variante, plus sophistiquée;
- la figure 7: illustre une variante de l'instrument dans laquelle celui-ci est solidaire du couvercle d'un flacon;
- la figure 8: illustre une variante de l'instrument dans laquelle le corps principal de celui-ci est pourvu d'une grille;
- la figure 9: illustre une variante de l'instrument dans laquelle l'embout est pourvu d'une grille.

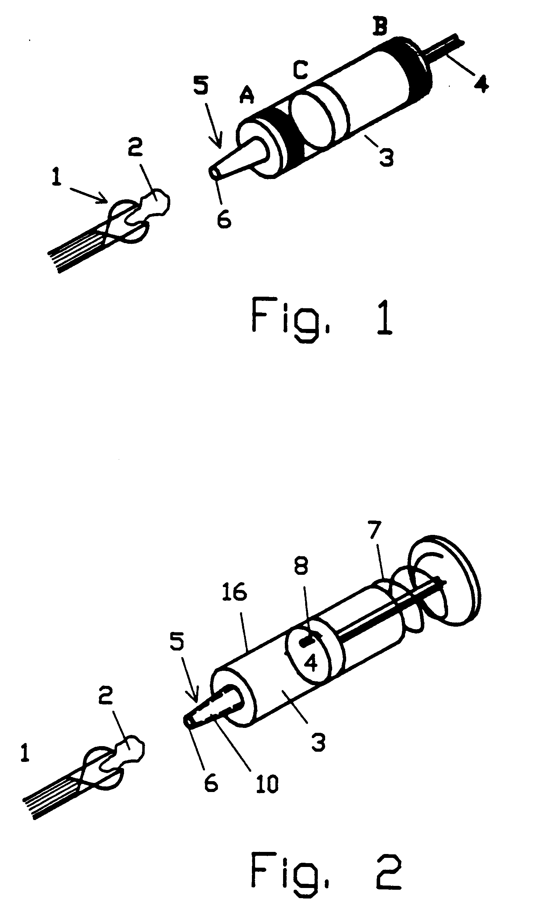

Sur la figure 1, on reconnaît une pince à biopsies 1, un prélèvement 2, une chambre volumique 3, un piston 4 et la partie de contact 5 entre le prélèvement et l'instrument sur laquelle on distingue l'orifice 6 du canal d'aspiration-éjection.

Dans un premier temps, le piston 4 est placé dans la position A qui indique l'état de moindre volume de la chambre volumique. Un moyen de rappel non représenté sur cette figure tend à repousser, ou retirer, le piston vers la position de volume maximum B. Il en résulte bien sûr un effet de succion et le prélèvement se colle à la partie de contact 5 et bouche l'orifice 6 du canal. Ce bouchage intervient plus ou moins vite, mais en général bien avant que le piston accède à la position B.

Pour fixer les idées on admet que le piston s'arrête dans la position intermédiaire C. En fait, si le contact entre le prélèvement et la partie de contact 5 est parfait dès le début, le bouchage est immédiat et l'intervalle entre la position A et la position C ne traduit que l'extensibilité de l'air contenu. Dans ce cas volontairement extrême, on comprend que si l'on exerce une pression sur le piston, le retour à la position A permettra à l'air contenu de reprendre son volume initial mais aucun jet d'air et aucun effet d'éjection n'en résultera. Le prélèvement, qui est collant, comme on l'a déjà dit, restera collé à la partie de contact 5. En fait, pour produire un jet d'air suffisant pour éjecter le prélèvement, il est nécessaire que le piston 4 puisse retrouver la position B du volume maximum.

Sur la figure 2, on reconnaît toujours la pince à biopsies 1, le prélèvement 2, la chambre volumique 3, le piston 4, la partie de contact 5 et l'orifice du canal 6. En outre on distingue le moyen de rappel 7, ici représenté par un ressort extérieur. Il convient de préciser, pour ne plus y revenir, que le ressort peut être placé à l'intérieur de la chambre volumique 3. Traversant de part en part le piston, un micro-canal 8 constitue une entrée d'air calibrée. Ce micro-canal peut aussi être aménagé en un autre point de l'instrument, notamment à proximité de la partie de contact, l'important étant que ce canal débouche à l'intérieur du volume enfermé dans la chambre volumique telle qu'elle se présente lorsque le piston est dans sa position de volume minimum.

Plaçant en regard les figures 1 et 2, on reprend le déroulement des opérations tel que décrit à propos de la figure 1. Lorsque le prélèvement 2 obstrue l'orifice 6 du canal de succion-éjection, le piston 4 ne s'arrête plus dans la position intermédiaire C, mais, sous l'action du moyen de rappel 7, il regagne bel et bien la position B du volume maximum grâce à l'apport de l'air qui transite par le micro-canal 8.

Lorsque le piston a regagné sa position de volume maximum, le prélèvement ne subit plus aucun effet de succion mais il adhère suffisamment à la partie de contact de l'instrument car il est collant.

La section du micro-canal est de première importance pour la réalisation de l'effet décrit. Si la section est trop grande, les effets de succion et d'éjection peuvent être amoindris dans une mesure qui rend l'instrument mal utilisable. Si la section est au contraire trop faible, le temps nécessaire pour que le piston regagne la position de volume maximum peut s'allonger considérablement et rendre fastidieuse l'utilisation de l'instrument. La section idéale doit être recherchée par tâtonnement car elle dépend des dimensions respectives que l'on choisit pour l'ensemble des parties de l'instrument.

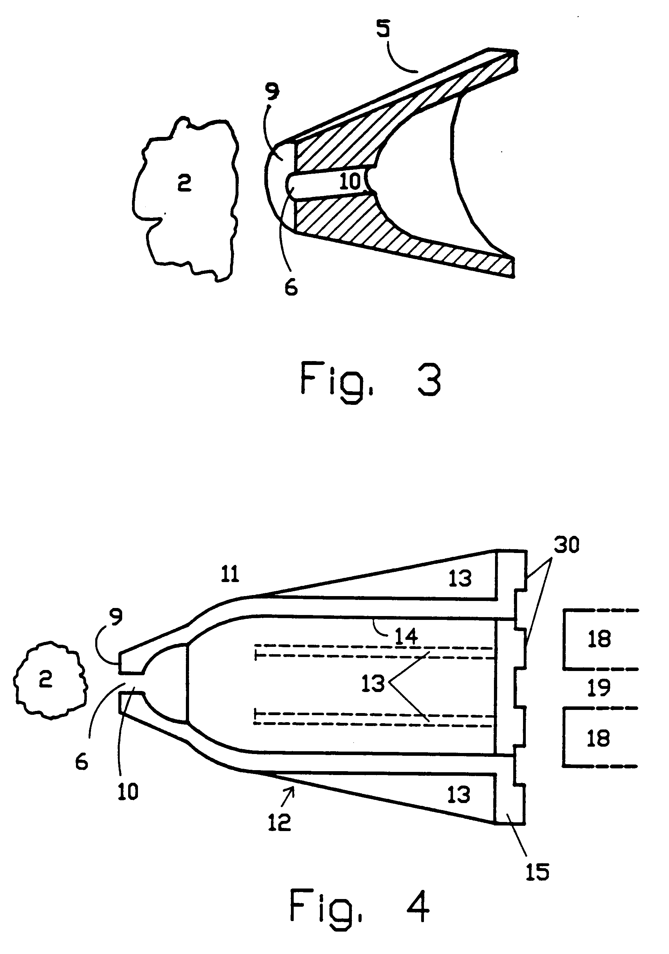

Sur la figure 3, on peut voir le prélèvement 2 ainsi que la partie de contact 5 de l'instrument. On distingue encore l'orifice 6 du canal de succion-éjection 10. Les proportions de la partie de contact 5 et plus particulièrement le rapport entre la surface de contact 9 et la section de l'orifice 6 du canal de succion-éjection sont importantes pour obtenir un bon compromis, une bonne balance, entre les divers effets de l'instrument. Le prélèvement étant collant, plus la surface de contact 9 est importante, plus le prélèvement adhérera à l'instrument. Cette surface peut être augmentée ou diminuée selon qu'on la façonne comme un pavillon ou selon un dôme ou un cône proéminents. D'une manière générale, les essais montrent que les meilleurs résultats sont obtenus lorsque la surface de contact 9 se trouve dans un plan normal à l'axe longitudinal de l'instrument.

Les prélèvements étant produits par des instruments divers, leur taille est bien sûr variable. Les précisions qui suivent sont données pour un prélèvement produit par une pince à biopsies utilisée en relation avec un fibroscope ou endoscope dont le canal de travail est de 1,8 mm de section. Pour ce type de prélèvements, il faut retenir, pour la surface de contact circulaire 9, un diamètre de 1 mm et, pour l'orifice 6 du canal, un diamètre de 0.5 mm. Ces dimensions donnent lieu à un rapport d'environ 3 à 1 entre la surface réelle de contact 9 et la section de l'orifice 6. Ces proportions peuvent être conservées dans la réalisation de parties de contact adaptées à des prélèvements plus volumineux comme à des prélèvements qui le seraient moins encore.

Il est recommandable de façonner la partie de contact 5 selon un cône qui fait la liaison entre la surface de contact 9 et le corps principal 16 de l'instrument, constitué pour l'essentiel de la chambre volumique. Un façonnage en forme de cône facilite en effet le positionnement de la partie de contact de l'instrument, notamment s'il s'agit d'introduire celle-ci entre les coupelles d'une pince à biopsies.

La partie de contact 5, dont les éléments essentiels viennent d'être décrits, est prévue pour pouvoir coopérer avec le reste de l'instrument selon trois modes. Ces trois modes dépendent d'une part de l'alternative concernant le caractère jetable (mono-usage) ou non jetable de l'instrument entier et d'autre part, si l'instrument est conçu pour être jetable, de la question de savoir si la partie de contact peut être prévue dans le moule d'injection du corps principal de l'instrument ou si au contraire cette partie de contact doit être rapportée sur un corps principal déjà formé.

Si l'instrument est jetable, il est possible d'utiliser les installations de fabrication des seringues plastiques telles qu'il en existe de nombreux modèles sur le marché. Dans ce cas, l'instrument se présente sensiblement tel qu'il est illustré à la figure 2, laquelle correspond à la première variante de l'instrument selon l'invention. Dans ce cas, si le moule d'injection peut être complété par l'empreinte de la partie de contact 5, il suffit de se rapporter aux caractéristiques décrites de la partie de contact et de conformer l'empreinte en conséquence.

S'il n'est en revanche pas possible d'introduire l'empreinte de la partie de contact dans le moule d'injection, il est recommandé de pourvoir la partie de contact d'un organe de liaison du type mâle-femelle qui pourra être enfiché à demeure dans l'organe correspondant du corps principal de l'instrument. A cet égard, il convient de noter que les seringues plastiques disposent toutes de tels aménagements en raison du fait qu'elles sont destinées à collaborer avec des aiguilles, qui portent les aménagements complémentaires.

Si l'instrument n'est pas conçu pour être jetable, la partie de contact comporte une zone d'accouplement correspondant au standard de connexion retenu pour la partie correspondante de l'instrument. La partie de contact se présente alors sous forme d'un embout amovible et est décrite ci-dessous en se référant à la figure 4.

Sur la figure 4, on découvre une coupe longitudinale de l'embout 11. L'embout 11 est réalisé par injection d'une matière qui peut être choisie par exemple parmi le PMMA-plexiglas, le polypropylène ou le Nylon. Dans tous les cas il est souhaitable, sinon fort recommandable, que la matière utilisée soit transparente car il est important de pouvoir vérifier par la vue si le prélèvement est ou non engagé dans l'embout sous l'action de l'instrument.

Il convient ici d'insister sur le fait que cette variante de l'instrument est conçue essentiellement dans le but que le prélèvement ne pénètre pas dans l'embout.

Outre les éléments décrits à propos de la figure 3, l'embout comporte également un tronc 12 sur lequel sont aménagées des ailettes extérieures 13. La fonction de ces ailettes est de donner des prises pour la manipulation de l'embout, en particulier lorsqu'il adapté sur le tronçon 18 du corps principal 16 destiné à le recevoir et lorsqu'il en est désolidarisé. En effet, des mouvements partiels de rotation en va-et-vient selon l'axe longitudinal de l'instrument sont utiles dans ces deux opérations. La cavité intérieur 14 de l'embout est conformée de manière à assurer une bonne coopération avec le tronçon 18 correspondant du corps principal 16. Finalement, l'embout se termine par une couronne 15 dont la forme sera commentée plus bas.

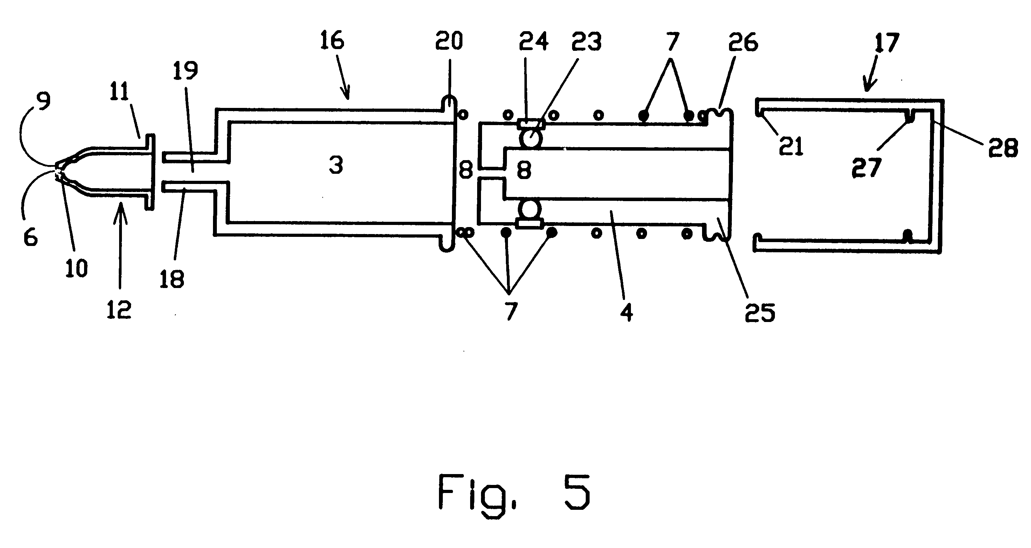

La figure 5 présente la première version non-jetable de l'instrument et l'on y retrouve bon nombre des éléments déjà décrits, soit l'embout 11 avec sa surface de contact 9, l'orifice 6 du canal de succion-éjection 10, le tronc de raccordement 12 avec sa cavité 14 et ses ailettes 13. Le piston 4 et son micro-canal 8 sont également présents. On retrouve aussi le moyen de rappel 7.

Dans cette variante on constate que l'instrument se compose essentiellement de cinq éléments, à savoir un embout 11, un corps principal 16, un piston 4, un ressort 7 et un capuchon 17. Le corps principal 16 a une forme qui rappelle celle de la partie principale d'une seringue et se présente donc comme un cylindre creux. Ce corps principal peut être réalisé en polypropylène, selon les techniques d'injection connues. Le corps principal a un diamètre extérieur d'environ 12 mm et un diamètre intérieur d'environ 10 mm. Sa longueur est d'environ 80 mm.

On remarque le tronçon de raccordement 18 qui est destiné à coopérer avec la cavité 14 de l'embout 11. Les dimensions de ce tronçon sont évidemment à mettre en relation avec celles que l'on choisit pour l'embout. Cependant l'expérimentation montre que l'on peut retenir une valeur de 3 mm pour le diamètre extérieur du tronçon 18, alors qu'un diamètre intérieur de 1 mm peut être choisi pour le canal 19. La longueur du tronçon de raccordement 18 est d'environ 8 mm. Le raccordement temporaire entre le tronçon 18 et l'embout 11 est réalisé au moyen d'un raccord conique usuel dans le domaine des instruments médicaux (système LUER-LOCK).

A l'extrémité du corps principal 16 opposée à l'embout 11, on remarque une lèvre annulaire 20 qui se déploie à l'extérieur du cylindre constituant essentiellement le corps principal 16. Cette lèvre annulaire assume deux fonctions distinctes. Elle sert de butée au ressort 7 et elle joue également le rôle de partie mâle destinée à coopérer avec la lèvre annulaire rentrante 21 du capuchon 17, qui, elle, joue le rôle de partie femelle. La coopération des lèvres annulaires 20 et 21 est destinée à solidariser le corps principal 16 et le capuchon 17 pour éviter que celui-ci ne soit éjecté par l'action du ressort 7. On peut souligner que l'ajustement du diamètre extérieur de la lèvre annulaire 20 et du diamètre intérieur du capuchon 17 est choisi de manière à tolérer le passage de l'air. Le piston 4, également réalisé en polypropylène, a des dimensions à mettre très normalement en relation avec celles du corps principal 16 avec lequel il doit coopérer. Il se présente sous forme d'un cylindre creux dont l'une des extrémités est close et forme la coupole. La longueur du piston 4 est d'environ 95 mm. Outre le micro-canal 8 que l'on a déjà abondamment décrit, le piston 4 comporte également un joint d'étanchéité 22. Ce joint d'étanchéité est constitué d'un joint torique "O-ring" 23 recouvert d'un anneau 24 en TEFLON (marque déposée). On a en effet pu constater qu'un joint "O-ring" seul n'était pas satisfaisant dans la mesure où l'amorce du mouvement est trop brutale, inconvénient que l'anneau en TEFLON atténue de manière tout à fait concluante, rendant l'amorce, de mouvement suffisamment douce.

A l'extrémité opposée à la coupole, le piston 4 comporte lui aussi une lèvre annulaire externe 25, dotée d'une gorge d'accrochage circulaire 26. La lèvre externe 25 assume deux fonctions distinctes. D'une part, elle sert de contre butée au ressort 7 et, d'autre part, elle joue, par sa gorge 26, le rôle de partie femelle destinée à coopérer avec la partie mâle que représente la lèvre interne 27 du capuchon 17, pour assurer la solidarisation du piston 4 et du capuchon 17 qui commande le piston 4. Finalement, le capuchon 17, dont les parties actives ont déjà été mentionnées, est aussi réalisé en polypropylène et se présente sous forme d'un cylindre creux terminé par un fond 28. Sa longueur est d'environ 40 mm.

On décrit maintenant le fonctionnement de l'instrument. Réalisé selon les indications de dimensions données plus haut, l'instrument a l'aspect et les proportions d'un stylo. L'opérateur adapte un embout sur le tronçon de raccord 18 et saisit l'instrument de la même manière qu'il saisirait un stylo à bille pour actionner le bouton poussoir avec son pouce. A l'aide de son pouce, l'opérateur appuie sur le fond 28 du capuchon 17. Solidaire du capuchon grâce à l'effet combiné de la gorge 26 et de la lèvre 27, le piston comprime le ressort 7 jusqu'à ce qu'il arrive en bout de course. Tout en maintenant l'instrument dans cette configuration, l'opérateur approche l'embout 11 du prélèvement jusqu'à ce que la surface de contact 9 touche le prélèvement. L'opérateur relâche alors la pression qu'il exerçait sur le fond du capuchon. Sous l'effet du ressort, le piston tend à reprendre sa position de repos. Il en résulte un effet d'aspiration qui fait adhérer le prélèvement à la surface de contact 9 par succion et l'opérateur en profite pour retirer immédiatement le prélèvement de l'instrument sur lequel ou dans lequel il se trouve. Sitôt que le prélèvement obstrue complètement l'orifice 6 du canal d'aspiration 10, le mouvement de retour du piston se voit en principe stoppé. C'est à ce moment qu'intervient le micro-canal 8 qui agit comme une entrée d'air calibrée. Le déficit de volume résultant de l'obstruction du canal d'aspiration 10 de l'embout est progressivement comblé par l'apport d'air transitant par le micro-canal 8. Le piston peut ainsi reprendre complètement sa position de repos. Comme on l'a dit en préambule, les prélèvements sont de petites masses collantes. Elles collent en fait suffisamment pour rester suspendues à l'embout, même si l'effet de succion initial diminue puis disparaît totalement pendant que le piston reprend sa position de repos. La deuxième partie de la manipulation peut alors être exécutée et l'opérateur place l'extrémité de l'instrument au-dessus du récipient et appuie à nouveau sur le capuchon à l'aide de son pouce. L'air enfermé à l'intérieur du corps principal est alors expulsé est le jet d'air qui en résulte à l'orifice 6 du canal d'aspiration 10 suffit largement à chasser le prélèvement, malgré la perte minime qu'autorise le micro-canal 8, et le prélèvement tombe dans le récipient de conservation.

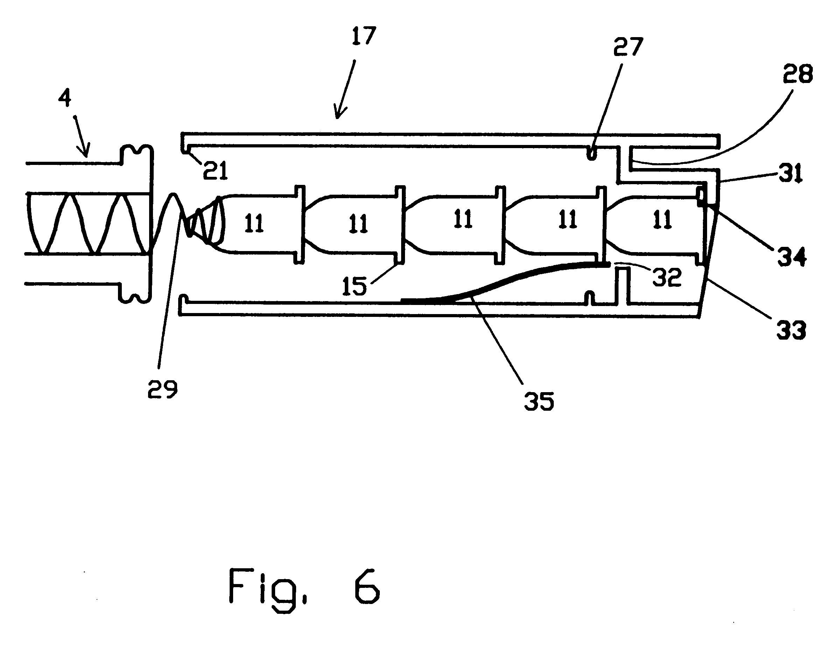

Au regard de la figure 6, qui représente une deuxième variante de l'instrument dans sa version à usage répétitif, il convient de se représenter l'instrument tel que décrit à la figure 5; le seul élément subissant des modifications étant le capuchon 17. Selon cette variante, le capuchon 17 est doté d'une réserve d'embouts 11, qui prend place dans le corps du piston 4. C'est d'ailleurs la raison pour laquelle on a décrit le piston 4 comme un cylindre fermé sur une extrémité à la figure 5; un piston tel qu'illustré à la figure 2 aurait tout aussi bien fait l'affaire.

On distingue encore sur la figure 6 un ressort 29 destiné à repousser la colonne des embouts 11 contre une languette de retenue en demi-lune 31. L'une des extrémités du ressort 29 prend appui sur la face intérieure de la coupole du piston 4. Son autre extrémité s'appuie sur le dernier embout de la colonne. Dans cette zone de contact, le diamètre du ressort 29 est resserré dans un premier temps puis reprend son diamètre original selon un cône qui épouse la forme générale de l'embout. Le fond 28 du capuchon comporte un orifice de sortie 32 qui débouche dans une niche cylindrique ovale 33. Cette niche se termine par la languette de retenue en demi-lune 31 qui comporte un rebord d'accrochage 34. Ce rebord est destiné à coopérer avec la couronne 15 de l'embout de manière à retenir ce dernier. Revenant très rapidement à la figure 4, on constate que la couronne de l'embout comporte des créneaux 30 qui sont prévus pour permettre à l'utilisateur d'avoir prise lorsqu'il dégage un embout avec son doigt en appuyant dessus, ce qui le libère du rebord d'accrochage 34. Ainsi libéré, l'embout sort de la réserve sous l'action du ressort 29. Une languette de maintien 35 repousse la colonne de manière que l'embout suivant vienne à son tour se mettre en position d'attente, la couronne 15 de l'embout suivant étant retenue par le rebord d'accrochage 34. La languette de maintien 35 permet d'éviter que le ressort 29 n'éjecte toute la colonne d'un coup.

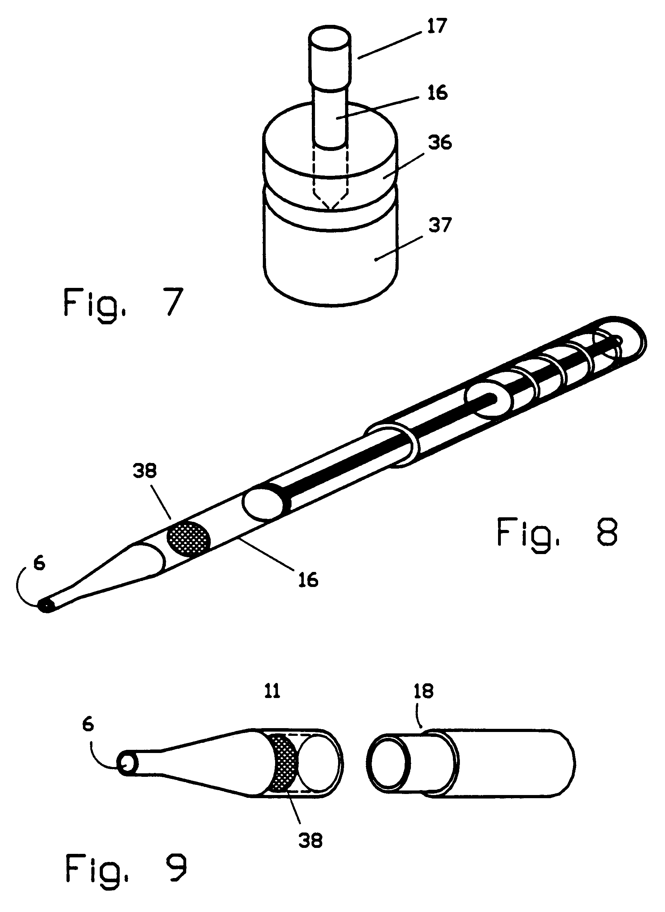

Selon une autre variante, représentée à la figure 7, l'instrument tel qu'il a été décrit, notamment sur les figures 1 à 5, est solidarisé avec le couvercle 36 du récipient 37 dans lequel le prélèvement doit être déposé. Outre cette particularité, cette variante se distingue des précédentes par le fait que d'une part il n'est plus nécessairement recherché que le prélèvement ne pénètre pas dans l'instrument et d'autre part il n'est plus forcément nécessaire non plus que la chambre volumique dispose d'un micro-canal d'entrée d'air calibrée. Les indications données précédemment au sujet des dimensions de la partie de contact de l'instrument avec le prélèvement et celles concernant le micro-canal ont moins d'importance dans cette variante.

Selon une autre variante encore, représentée aux figures 8 et 9, l'instrument est pourvu d'une grille transversale fine 38, réalisée par exemple en fibre de carbone, et dont le but est d'empêcher que le prélèvement ne pénètre dans l'instrument au delà de la position fixée par la grille. Dans cette variante, on suppose que la section de l'orifice 6 est choisie de manière que le prélèvement puisse passer au travers de l'orifice et s'engager dans l'instrument. A la figure 8, la grille est positionnée dans le corps principal 16 de l'instrument. Cette variante suppose qu'un instrument est utilisé pour chaque prélèvement et que l'instrument entier est envoyé au laboratoire d'analyses. A la figure 9, la grille est positionnée dans l'embout de sorte que c'est uniquement les embouts qui sont envoyés au laboratoire.

Les avantages de l'instrument selon l'invention sont de faciliter la manipulation des prélèvements tout en ménageant ceux-ci mieux que ce n'était le cas jusqu'ici et en ménageant de la même façon les instruments utilisés pour effectuer ces prélèvements. De plus, la possibilité de réaliser l'instrument sous forme jetable permet d'utiliser des installations de fabrication et d'injection existantes qui ont des capacités de production énormes et rendent le coût de fabrication de l'instrument aussi bas que celui des seringues courantes. Dans sa version non-jetable, l'adoption des matériaux décrits permet la stérilisation de l'instrument et offre, en particulier grâce à un joint d'étanchéité très doux, un confort d'utilisation appréciable. Finalement, les prélèvements sont réalisés par des médecins spécialisés au moyen d'appareils ou d'instruments extrêmement sophistiqués et coûteux; les moyens d'analyse sont aussi à la pointe de la technique, mais l'étape incontournable qui consiste à manipuler le prélèvement est laissée au "système D". Le transfert pneumatique des prélèvements, qui est rendu possible par l'instrument selon l'invention, remédie à cette faiblesse anachronique et permet d'éviter que des appareils précieux restent immobilisés ou soient endommagés en raison d'une opération de simple manutention, souvent malaisée mais toujours indispensable.

## Revendications

1. Instrument pour la manutention de biopsies et de prélèvements comportant un corps principal (16), à l'intérieur duquel se trouve un piston (4) permettant de faire varier le volume enfermé; un moyen de rappel (7) tendant à ramener le piston dans une position de repos correspondant au volume enfermé maximum, l'instrument comportant une partie (5) destinée au contact avec le prélèvement, ladite partie présentant un canal de succion - éjection, caractérisé par le fait qu'il comporte un micro-canal (8) mettant en communication permanente le volume enfermé avec l'atmosphère et constituant un passage d'air calibré de l'intérieur vers l'extérieur de l'instrument et vice-versa.

2. Instrument selon la revendication 1, caractérisé par le fait que le micro-canal (8) réalise la mise en communication entre le volume enfermé et l'atmosphère alors que le volume enfermé est à son minimum.

3. Instrument selon l'une des revendications 1 et 2, caractérisé par le fait que le micro-canal (8) est pratiqué au travers de la coupole du piston (4).

4. Instrument selon la revendication 1, caractérisé par le fait que le corps principal (16) comporte une grille transversale fine (38) destinée à empêcher que le prélèvement pénètre dans le corps principal au-delà de la position de la grille.

5. Instrument selon la revendication 1 caractérisé, par le fait qu'il est rendu solidaire du couvercle (36) d'un flacon (37).

6. Instrument selon la revendication 1, caractérisé par le fait que le canal de succion-éjection (10) débouche dans une surface de contact (9), l'importance de la surface de contact (9) étant plus grande que la section de l'orifice (6) du canal (10).

7. Instrument selon la revendication 6, caractérisé par le fait que la surface de contact (9) et la surface de l'orifice (6) se trouvent dans un rapport de trois à un.

8. Instrument selon la revendication 6, caractérisé par le fait que la surface de contact (9) du canal de succion-éjection (10) se trouve dans un plan normal à l'axe longitudinal de l'instrument.

9. Instrument selon la revendication 1, caractérisé par le fait que la partie de contact (5) du canal de succion-éjection (10) a une zone terminale conformée selon un cône.

10. Instrument selon la revendication 1, caractérisé en ce que la partie de contact (5) se présente sous forme d'un embout amovible (11) qui coopère avec un tronçon de raccordement (18).

11. Instrument selon la revendication 10, caractérisé en ce que l'embout (11) possède une grille transversale fine (38) destinée à empêcher que le prélèvement pénètre dans l'embout au-delà de la position de la grille.

12. Instrument selon l'une des revendications 10 et 11, caractérisé par le fait que l'embout (11) est pourvu d'ailettes extérieures (13).

13. Instrument selon l'une des revendications 10 à 12, caractérisé par le fait que l'embout (11) possède une couronne (15) pourvue de créneaux (30).

14. Instrument selon la revendication 1, caractérisé par le fait que l'extrémité de commande du piston (4) est pourvue d'un capuchon (17) qui recouvre partiellement le corps principal (16) de l'instrument.

15. Instrument selon l'une des revendications précédentes, caractérisé en ce que le piston (4) se présente sous forme d'un cylindre creux dont l'une des extrémités est close et constitue la coupole du piston.

16. Instrument selon la revendication 15, caractérisé en ce que l'intérieur du piston (4) constitue un logement dans lequel prend place un ou plusieurs embouts.

17. Instrument selon l'une des revendications 13 et 16, caractérisé par le fait que le capuchon (17) est pourvu d'un orifice (32) débouchant dans une niche (33) partiellement fermée par une languette en demi-lune (31) possédant un rebord d'accrochage (34) destiné à coopérer avec la couronne (15) de l'embout (11).

18. Instrument selon la revendication 17, caractérisé en ce qu'un ressort (29) prend appui d'une part sur la face intérieure de la coupole du piston (4) et d'autre part contre un embout (11) et en ce qu'une languette de maintien (35) repousse l'embout de sorte que celui-ci est retenu par une languette en demi-lune (31) et par un rebord d'accrochage (34), évitant ainsi l'éjection involontaire de l'embout (11).

## Claims

1. Instrument for the handling of biopsies and specimens comprising a main body (16), within which is disposed a piston (4) enabling to vary the volume enclosed; a means of recoil (7) which tends to bring the piston back into a position of rest corresponding with the maximum volume enclosed, the instrument including a portion (5) intended for contact with the specimen, said portion having a channel for suction-ejection, characterised in that it comprises a micro-channel (8) permanently communicating the volume enclosed with the atmosphere and providing a calibrated flow of air into and out of the instrument.

2. Instrument according to claim 1, characterised in that the micro-channel (8) communicates the volume enclosed with the atmosphere when said volume enclosed is at its minimum.

3. Instrument according to one of claims 1 and 2, characterised in that the micro-channel (8) is made through the head of the piston (4).

4. Instrument according to claim 1, characterised in that the main body (16) comprises a fine transverse grating (38) intended for preventing the specimen from penetrating into the main body beyond the position of the grating.

5. Instrument according to claim 1, characterised in that the same is made integral with the cap (36) of a bottle (37).

6. Instrument according to claim 1, characterised in that the channel for suction-ejection (10) ends as an orifice within a contact area (9), the surface of said contact area being larger than the surface of the orifice (6) of the channel (10).

7. Instrument according to claim 6, characterised in that the surface of the contact area (9) and the surface of the orifice (6) are in a ratio of the order of 3 to 1.

8. Instrument according to claim 6, characterised in that the contact area (9) of the channel for suction-ejection (10) lies in a plane normal to the longitudinal axis of the instrument.

9. Instrument according to claim 1, characterised in that the contact portion (5) of the channel for suction-ejection (10) has a terminal zone which is shaped in a cone.

10. Instrument according to claim 1, characterised in that the contact portion (5) is made in the form of a removable tip (11) which cooperates with a connector section (18).

11. Instrument according to claim 10, characterised in that the tip (11) has a fine transverse grating (38) intended for preventing the specimen from penetrating into the tip beyond the position of the grating.

12. Instrument according to one of claims 10 and 11, characterised in that the tip (11) is provided with external fins (13).

13. Instrument according to one of claims 10 to 12, characterised in that the tip (11) has a crown (15) having castellations (30).

14. Instrument according to claim 1, characterised in that the operating end of the piston (4) is provided with a hood (17) which partially covers the main body (16) of the instrument.

15. Instrument according to one of the preceding claims, characterised in that the piston (4) is made in the form of a hollow cylinder of which one end is closed and forms the head of the piston.

16. Instrument according to claim 15, characterised in that the inside of the piston (4) forms a housing in which take place one or more tips.

17. Instrument according to one of the claims 13 and 16, characterised in that the hood (17) is provided with an aperture (32) opening into a niche (33) which is partially closed by a small halfmoon tongue (31) having a catch edge (34) intended for cooperating with the crown (15) of the tip (11).

18. Instrument according to claim 17, characterised in that a spring (29) bears on the one hand against the inner face of the head of the piston (4) and on the other hand against a tip (11) and in that a retainer tongue (35) pushes over the tip so that the same is retained by a halfmoon tongue (31) and by a catch edge (34), thus avoiding the involuntary ejection of the tip (11).

## Patentansprüche

1. Vorrichtung zur Handhabung von Biopsien und Proben, mit einem Hauptgehäuse (16), in dessen Innern sich ein Kolben (4) befindet, der eine Veränderung des eingeschlossenen Volumens gestattet; einer Rückstelleinrichtung (7), die dazu neigt, den Kolben in eine Ruhestellung zurückzubringen, die dem maximalen eingeschlossenen Volumen entspricht, wobei die Vorrichtung einen Teil (5) aufweist, der für den Kontakt mit der Probe bestimmt ist, wobei der Teil einen Saug-Ausstoß-Kanal aufweist, dadurch gekennzeichnet, daß sie einen Mikro-Kanal (8) aufweist, der das eingeschlossene Volumen ständig mit der Atmospäre in Verbindung hält und einen kalibrierten Luftdurchtritt vom Innern der Vorrichtung nach außen und umgekehrt bildet.

2. Vorrichtung nach Anspruch 1, dadurch gekennzeichnet, daß der Mikro-Kanal (8) die Herstellung der Verbindung zwischen dem eingeschlossenen Volumen und der Atmosphäre verwirklicht, wenn das eingeschlossenen Volumen bei seinem Minimum ist.

3. Vorrichtung nach einem der Ansprüche 1 und 2, dadurch gekennzeichnet, daß der Mikro-Kanal (8) durch die Haube des Kolbens (4) hindurch ausgebildet ist.

4. Vorrichtung nach Anspruch 1, dadurch gekennzeichnet, daß das Hauptgehäuse (16) ein feines Gitter (18) in Querrichtung aufweist, das dazu bestimmt ist, zu verhindern, daß die Probe in das Hauptgehäuse bis über die Position des Gitters hinaus eindringt.

5. Vorrichtung nach Anspruch 1, dadurch gekennzeichnet, daß sie mit dem Deckel (36) eines Glasfläschchens (37) einstückig ist.

6. Vorrichtung nach Anspruch 1, dadurch gekennzeichnet, daß der Saug-Ausstoß-Kanal (10) in eine Kontaktfläche (9) mündet, wobei die Ausdehnung der Kontaktfläche (9) größer als die Schnittfläche der Öffnung (6) des Kanals (10) ist.

7. Vorrichtung nach Anspruch 6, dadurch gekennzeichnet, daß die Kontaktfläche (9) und die Fläche der Öffnung (6) zueinander in einem Verhältnis von drei zu eins stehen.

8. Vorrichtung nach Anspruch 6, dadurch gekennzeichnet, daß sich die Kontaktfläche (9) des Saug-Ausstoß-Kanals (10) in einer normal zur Längsachse der Vorrichtung verlaufenden Ebene befindet.

9. Vorrichtung nach Anspruch 1, dadurch gekennzeichnet, daß der Kontaktteil (5) des Saug-Ausstoß-Kanals (10) einen in Form eines Kegels ausgebildeten Endbereich hat.

10. Vorrichtung nach Anspruch 1, dadurch gekennzeichnet, daß der Kontaktteil (5) die Form eines abnehmbaren Ansatzstücks (11) hat, das mit einem Verbindungsteilabschnitt (18) zusammenwirkt.

11. Vorrichtung nach Anspruch 10, dadurch gekennzeichnet, daß das Ansatzstück (11) ein feines Gitter (38) in Querrichtung besitzt, das dazu bestimmt ist, zu verhindern, daß die Probe in das Hauptgehäuse bis über die Position des Gitters hinaus eindringt.

12. Vorrichtung nach einem der Ansprüche 10 und 11, dadurch gekennzeichnet, daß das Ansatzstück (11) mit Außenrippen (13) versehen ist.

13. Vorrichtung nach einem der Ansprüche 10 bis 12, dadurch gekennzeichnet, daß das Ansatzstück (11) eine mit Scharten (30) versehene Krone (15) besitzt.

14. Vorrichtung nach Anspruch 1, dadurch gekennzeichnet, daß das Antriebsende des Kolbens (4) mit einer Kappe (17) versehen ist, die das Hauptgehäuse (16) der Vorrichtung partiell abdeckt.

15. Vorrichtung nach einem der vorhergehenden Ansprüche, dadurch gekennzeichnet, daß der Kolben (4) die Form eines Hohlzylinders darstellt, bei dem eines der Enden geschlossen ist und die Haube des Kolbens bildet.

16. Vorrichtung nach Anspruch 15, dadurch gekennzeichnet, daß das Innere des Kolbens (4) einen Sitz bildet, in dem ein oder mehrere Ansatzstücke sitzen.

17. Vorrichtung nach einem der Ansprüche 13 und 16, dadurch gekennzeichnet, daß die Kappe (17) mit einer Öffnung (32) versehen ist, die in eine Nische (33) mündet, die mittels einer halbmondförmigen Federzunge (31) partiell geschlossen ist, welche eine Aufhängungsleiste (34) besitzt, die dazu bestimmt ist, mit der Krone (15) des Ansatzstückes (11) zusammenzuwirken.

18. Vorrichtung nach Anspruch 17, dadurch gekennzeichnet, daß eine Feder (29) einerseits gegen die Innenfläche der Haube des Kolbens (4) und andererseits gegen ein Ansatzstück (11) drückt und daß eine Halteblattfeder (35) das Ansatzstück derart verschiebt, daß es von einer halbmondförmigen Federzunge (31) und einer Aufhängungsleiste (34) gehalten wird, wodurch ein ungewolltes Ausstoßen des Ansatzstückes (11) verhindert wird.
